# EUROPEAN PATENT APPLICATION

(11) **EP 0 978 564 A1**
(43) Date of publication of application: **09.02.2000**
(21) Application number: 98916797.8
(22) Date of filing: 27.04.1998
(51) Int. Cl.: C12N 15/62, C12N 15/31, C07K 14/245, G01N 33/555, C07K 14/16

(54) **RECOMBINANT FUSION PROTEINES BASED ON BACTERIAL ADHESINES FOR DIAGNOSTIC ASSAYS**

(30) Priority: 28.04.1997 CU 4797
(71) Applicant: Entro De Ingenieria Genetica Y Biotecnologia (CIG B), Ciudad Habana 10600 (CU)
(72) Inventor: DUENAS PORTO, Marta, G., Playa Ciudad Habana 10600 (CU); AYALA AVILA, Marta, Playa Ciudad Habana 10600 (CU); GAVILONDO COWLEY, Jorge V, Ciudad Habana 10400 (CU); FREYRE ALMEIDA, Freya M., Playa Ciudad Habana 10600 (CU); BELL GARC A, Hansell, Playa Ciudad Habana 12100 (CU)
(74) Representative: Ottevangers, Sietse Ulbe
(86) International application number: CU9800005
(87) International publication number: WO9849327

(57) **Abstract**

The present invention relates to the field of biotechnology, particularly to the development of recombinant fusion proteines based on bacterial adhesines and their use in diagnostic assays of hemagglutination for the specific detection of antibodies or antigenes in the blood and other biological samples. The object from a technical point of view is to obtain recombinant fusion proteines, or biochemical conjugates, useful for the development of simple diagnostic assay sytems based on bacterial adhesines or their domains of molecular interaction with red cells. The use of adhesion proteines of *E. coli* as a base of fusion recombinant proteines with an activity of union to human erythrocytes enhances the characteristics of expression in said host thereby eliminating potential problems in production yields associated to bacterial toxicity.

## Description

### Technical branch

The present invention is related to the field of biotechnology and in particular with the development of recombinant fusion proteins based on bacterial adhesins and their use in haemagglutination diagnostic assays for the specific detection of antibodies or antigens in blood and other biological samples.

### Previous technique

Enteropathogenic bacterial strains frequently posses surface proteins which act as adhesion factors (hereafter denominated "adhesins"), and allow bacteria to adhere to the host cells (Gaastra, W. y de Graaf, F.K. 1982. Microbiol. Rev. 46. 129-161). In some cases, such as that of strain K88, the adhesive activity is associated with a predominant protein subunit present in polymeric rod-like structures (denominated hereafter and indistinctively as fimbriae, and pilus-pili) (Jacobs, A.A., Venema, J., Leevan, R., van Pelt-Heers-Chap, H. y de Graaf, F.K. 1987. 169. 735-741). In other cases, such as that of the Pap fimbriae, this structure functions as support, bearing the adhesive elements at its tip (Lindberg, F., Lund, B., Johanssen, L. y Normark, S. 1987. Nature. 328. 84-87). In types I and X fimbriae the subunits responsible for adhesion are also the products of genes different from that encoding for the major subunit of the pilus (Maurer, L. y Orndorff, P.E. 1987. J. Bacteriol. 169. 640-645, Hacker, J., Schmitd, G., Hughes, S., Knapp, S., Marget, M. y Goebel, W. 1985. Infect. Immun. 47. 434-440).

Bacterial adhesins are not invariably assembled into pilus structures. Many adhesins cannot be visualized by standard electron microscopy techniques, and are thus considered to be non-pilus adhesins. The architecture of non-pilus adhesins is not well known, but most are presumably linked to the bacterial cell surface as monomers or simple oligomers.

In nature, many microbes can thrive in a variety of ecological niches, whereas others are restricted to a specific microenvironment. The recognition of host cell receptors by both pilus and non-pilus adhesins is an extremely fine tuned process, that originates a very selective interaction.

The Gram-negative bacterium *Escherichia coli* (E. coli) can colonize the intestinal tracts of many mammals and other vertebrates and cause a number of different infections in its host. The adhesive pili from Gram-negative bacteria generally adopt two basic morphologies: (a) rod-like fibers with a diameter of approximately 7 nm, or (b) flexible, thin fibrilliae with a diameter of 2-5 nm (de Graaf, F. K. y Mooi, F. R. 1986. Adv. Microb. Phyisiol. 28. 65-143). For example, type 1 pili are rod-like fibers that bind mannose, and P pili are rigid helicoidal polymers () that bind the alpha-D-galactopyranosyl-(1-4)-beta-D-galactopyranoside (Gal(1-4)Gal) moiety present in the globoseries of glycolipids on cells lining the upper urinary tract (Bock, K., Breimer, M., Hansson, G. C., Karlsson, K.A., Larson, G., Leffer, H., Samuelsson, B. E., Stomberg, N., Svanborg-Eden, C. y Turin, J. J. Biol. Chem. 260. 8545- 8551, Leffer, H. y Svanborg-Eden, C. 1980. FEMS Microbiol. Lett. 8. 127- 134, Stromberg, N., Nyholm, P.G., Pasher, I. y Normark, S. 1991. PNAS USA. 88. 9340- 9344). In contrast, other pili, such as those facilitating intestinal colonization, and those associated with enterotoxin-producing E. coli, form an open helical structure without an axial hole and are, consequently, much thinner and less rigid structures (de Graaf, F. K. y Mooi, F. R. 1986. Adv. Microb. Phyisiol. 28. 65-143, 1 Stromberg, N., Nyholm, P.G., Pasher, I. y Normark, S. 1991. PNAS USA. 88. 9340-9344).

Genes involved in the biosynthesis and expression of functional P pili are clustered in an operon structure in the bacterial chromosome of about 5-10% of human fecal E. coli isolates and up to 90% of the strains isolated from the urinary tract of children with acute pyelonephrittis (Hull, R.A., Gill, R.E., Hsu, P., Minshaw, B.H. y Falkow, S. 1981. Infect. Immun. 33. 933-938, Lund, B., Marklund, B.I., Stromberg, N., Lindberg, F., Karlsson, K. A. y Normark, S. 1988. Nature. 328. 84-87, Marlund, B.I., Tennet, J.M., Garcia, E., Hamers, A., Baga, M., Lindberg, F., Gaastra, W. y Normark, S. 1992. Mol. Microbiol. 6. 2225-2242, Plos, K., Carter, T., Hull, S., Hull, R. y Svanborg-Eden, C. 1990. J. Infect. Dis. 163. 549- 558). The structure of the entire pap gene cluster has been determined and has been shown to encode 11 genes, each of which has been studied by extensive mutant analysis (Figure 1).

The PapG adhesin is seemingly a two-domain molecule. Purification studies of N-terminal truncated variants of PapG using Gal(1-4)Gal affinity chromatography have shown that the N-terminal domain of this molecule contains the receptor-binding site (1 Hultgren, S., Lindberg, F., Magnusson, G., Kihlberg, J., Tennent, J.M. y Normark, S. 1989. Proc. Natl. Acad. Sci. USA. 86. 4357- 4361). In addition, fusion of the 5' end of the papG gene encoding the receptor-binding domain, to the gene encoding the maltose-binding protein, results in a soluble product that binds both maltose and Gal(1-4)Gal.

The C-terminal domain of PapG contains a recognition surface for the periplasmic PapD chaperone protein. The chaperone-adhesin interaction is a prerequisite for the presentation of the adhesin on the microbial surface, allowing its assembly in the pilus, and making the receptor binding domain accessible for the recognition of eukaryotic receptors. The chaperone binds to the C-terminal assembly domain of PapG in a manner that does not interfere with the receptor binding activity of the N-terminal domain ( Hultgren, S., Lindberg, F., Magnusson, G., Kihlberg, J., Tennent, J.M. y Normark, S. 1989. Proc. Natl. Acad. Sci. USA. 86. 4357- 4361). PapG possesses virtually the same galabiose binding specificity and affinity, both in its pre-assembly state bound to PapD, and when present at the pilus tip (Hultgren, S., Lindberg, F., Magnussen, G., Kihlberg, J., Tennent, J.M. y Normark, S. 1989. Proc. Natl. Acad. Sci. USA. 86. 4357- 4361).

The binding between pyelonephritic E. coli and human P erythrocytes has been investigated using various receptors analogues (Kilhberg, J., Hultgren, S.J., Normark, S. y Magnusson, G. 1989. J. Am. Chem. Soc. 111. 6364-6368). The beta-galabiose portion of the erythrocyte glycolipids was found to bind to the pilus tip PapG adhesin by hydrogen bonds directed toward five oxygen□ atoms located on an edge of the disaccharide, and by hydrophobic interactions with the non-polar cavity of the adhesin (Kilhberg, J., Hultgren, S.J., Normark, S. y Magnusson, G. 1989. J. Am. Chem. Soc. 111. 6364-6368).

The F41 is an adhesin derived from E. coli serogroup O, associated with diarrhea in lambs and calves (enterotoxigenic E. coli, ETEC). This adhesin provides bacteria with an antigenic and hemagglutinating surface structure which causes adherence to interstitial cells (Chien, S. 1975. En: D.M. Surgenor (ed.). The Red Blood Cell, vol. 2. Acad. Press, Inc. New York., Morris, J. A., Thorns, C. J., Scott, A.C., Sojka, W.J. y Wells, G.A. 1983. Infec. Immun. 36. 1146-1153, Morris, J. A., Thorns, C. J., Scott, A.C., Sojka, W.J. y Wells, G.A. 1983. J. Gen. Microbiol. 129. 2753-2759). This antigen has a subunit of an approximate molecular weight (Mw) of 29,500 daltons (Da), and has shown to be protective in the infant mouse model, and in the vaccination of piglets ().

Glycophorin A (GFA), the major human erythrocyte membrane glycoprotein, was shown to be the receptor for uropathogenic IM11165 E. coli, and for those displaying the F41 adhesin (Jokinen, M., Ehnholm, C.,. Visnen-Rhen, V., Korhonen, T., Pipkoru, R., Kalkkinen, N y Gamberg, C. G. 1985. Eur. J. Biochem. 147. 47-52, Vaisanen, V., Korhonen, T., Jokinen, M., Gamberg, C.G. y Ehnholm, C. 1982. Lancet i. 1192). GFA belongs to a group of sialoglycopeptides found in human erythrocytes.

GFA is the major representative of this group (about 75% of the total), and is composed by a single polypeptide of 131 amino acids, and of carbohydrates that make up to 60% of its molecular mass. The N-terminal 70 aminoacids protrude from the membrane lipid bilayer, and 16 of these are linked to carbohydrates. The binding for F41 was shown to be shear-enhanced towards the M blood type. The M or N blood type polymorphism is known to lie in the first and fifth amino acids of the NH2 terminus of the GFA peptide chain (Astee, D. J. 1981. Sem. Hematol. 18. 13-31).

In MM glycophorin the N terminus is a serine, and glycine occupies position five, while the NN glycophorin has leucine and glutamic acid, respectively, in these locations. GFA isolated from individuals with the MN blood type contains equimolar mixtures of each specie. In all cases, the second, third, and fourth amino acids are O-glycosilated with a tetrasaccharide bearing two residues of N-acetyl neuraminic acid (NANA). These oligosaccharides inhibit the binding to GFA, but NANA is not involved in the binding.

In contrast to most other known adhesins of ETEC, which are plasmid encoded, F41 is chromosomally encoded. The genetic determinants for F41 have been cloned and expressed in E. coli K12, and contain a 86 kDa porin, an accessory protein of 29 kDa that may act as a periplasmic chaperone, and the 29 kDa F41 antigen. This genetic organization is similar to that reported for other fimbrial adhesins such as K88, and the P pilus.

There are many agglutination diagnostic systems in the market, for a variety of diseases and states. Most employ latex particles, coated with antigens or antibodies, according to the specific nature of the diagnostic to be made. Hemagglutination, a very old diagnostic technique, is also still used due to its feasibility and simplicity.

Very recently, a novel type of assay that uses the self-agglutination of red blood cells from the tested individuals has been developed. The basis of this test is a bivalent molecule, composed by an antibody (or one of its binding sites) specific for erythrocytes, and by an antigenic moiety of a human infectious agent. If molecules as such are placed in contact with a small sample of blood from a seropositive individual, the erythrocytes will agglutinate in seconds because anti-antigen antibodies bridge the fusion proteins attached by their antibody binding site(s) to the red cells. In a sample from a seronegative individual no bridging occurs and hence, no hemagglutination.

The concept of self-agglutination of erythrocytes from a blood sample, using an antibody specific to GFA conjugated to diagnostic peptides, has been developed for HIV testing and is the basis of a commercial diagnostic test (SimpliRed. CSIRO. Australia).

The "first generation version" of this self-agglutination reagent, made by biochemical coupling of an antibody to GFA and HIV-1 peptides, has been followed-up by work from the same group using recombinant Fab and single chain Fv (scFv) antibody fragments specific for GFA, genetically fused to sequences encoding for HIV-1 and HIV-2 diagnostic peptides (Lilley, G., Dolezal, O., Hillyard, C.J., Bernard, C y Hudson, P.J. 1994. J. Immuno. Meth. 171 (2), 211-226).

This approach theoretically avoids difficulties related to biochemical conjugation. In a recent conference Lilley and associates (Lilley, G., Dolezal, O., Hillyard, C.J., Bernard, C y Hudson, P.J. 1994. J. Immuno. Meth. 171 (2), 211-226) reported the successful production of such fusion proteins, and showed data suggestive of an improved diagnostic performance of these molecules when compared to the biochemical conjugates. This group has applied for patents in Europe, Australia, Unites States of America for the described bifunctional recombinant proteins, specifically directed to targets as HIV, HBsAh, the D dimer, and a canine antigen (Lilley, G. et al. (1993) Reagent for agglutination assays. *International Publication Number:* WO 93/24630. Diciembre 9).

### DETAILED DISCLOSURE OF THE INVENTION

For the construction of the adhesin-peptide fusion proteins the total DNA content of piliated bacteria was first extracted.

These DNAs were use as templates for specific amplification reactions of the genes encoding the F41 and the Pap G proteins (specific for human GFA, and the P red blood antigen, respectively). In these experiments we used the polymerase chain reaction (PCR) technique and a set of synthetic oligonucleotides designed on the basis of published DNA sequences for these two proteins.

The amplified genes were cloned into bacterial three different expression vectors, and one yeast expression vector, together with a 3'-end sequence encoding for a HIV-1 gp41 diagnostic peptide. The 16 aminoacid peptide sequence was first assembled in vitro from two complementary synthetic oligonucleotides, and then ligated to the cloned PCR products. The first two bacterial expression vectors, developed in our laboratories and denominated pPACIB.7+ and pPACIB.9+, contain an operon comprising an inducible Tryptophan (tryp) promoter, a signal peptide (ompA) for periplasmic secretion in pPACIB.7+, a 26 aminoacid fragment of Human Interleukin 2 for high levels of intracelular expression on pPACIB.9+, six-histidine sequences for Immobilized Metal Affinity Chromatography (IMAC) purification, and a transcription terminator of the T4 phage, all in a pBR322 base. The third bacterial plasmid employed in our experiments was the pQE 60 vector from Qiagen, with the phage T5 promoter and 2 lac operators, a synthetic ribosomal binding site, a 3' histidine coding sequence, and two strong transcriptional terminators, on a pBR322 base.

All the aforementioned genetic constructions, ligated to the correspondent bacterial expression vector, were used to transform XL-1 blue competent cells from Stratagene. The colonies that grew on selective solid medium were evaluated by restriction enzyme digestion analysis. Plasmid DNA extracted from selected clones was sequenced.

The selected plasmids, denominated pPAP-HIVs and pF41-HIVs for the soluble periplasmic expression, and pPAP-HIVi and pF41-HIVi for expression in cytoplasm, were used to transform different E. coli strains for expression studies.

New soluble proteins, with approximate sizes of 35 kDa (for pPAP-HIV1s) and 31 kDa (for pF41-HIV1s) in SDS polyacrylamide gel electrophoresis (SDS-PAGE), were recovered from the culture supernatant and the periplasmic fractions of cells transformed with the pPACIB.7+ derived plasmids. Western blot studies with specific antisera showed that these proteins corresponded to the fusion of the Pap G and F41 adhesins to the HIV-1 peptide.

The study of expression of the fusion proteins in the bacterial cytoplasm, using pPACIB.9+ and pQE 60 as vectors, showed high levels of intracellular new proteins with the apparent molecular weights expected for the fusion proteins, in SDS-PAGE. These proteins were specifically recognized in Western blot with antisera against the adhesins, and to the HIV-1 peptide.

The recombinant fusion proteins were extracted from the periplasmic fractions using repeated cycles of freezing and thawing, or from the bacterial cytoplasm by sonication and solubilizing agents. The four fusion proteins were purified by IMAC using Cu+2 or Ni+2 as the transition metals and a pH gradient for elution. In vitro refolding of the two fusion proteins originated in the bacterial cytoplasm was performed by controlled dialysis in redox buffers.

In the case of the yeast expression strategy the gene inserts encoding for both fusion proteins were cloned in the pHIL-S1 Pichia pastoris vectors from InVitrogen. Recombinant clones identified in bacterial cells were used to prepare plasmid DNA that was linearized, electroporated in Pichia pastoris, and the cells seeded in adequate selection medium. Positive recombinant yeast clones were identified by colony PCR to check integration, and submitted to induction procedures. Cells and culture supernatant were screened by SDS-PAGE and Western blot for expression of the two fusion proteins.

The four soluble purified bacterial fusion proteins, and the two unpurified yeast fusion proteins were tested for agglutination with suspensions of O+ human erythrocytes spiked with: (a) an specific antiserum against each adhesin, (b) a human serum containing antibodies specific for the HIV-1 peptide, or (c) an unrelated antiserum. The recombinant fusion proteins visibly agglutinated erythrocytes in the red blood cell samples containing the antiserum specific against the adhesin in question, and the human serum specific for the peptide.

Finally, the six fusion proteins were assayed for agglutination of direct blood samples from VIH-1 seropositive and seronegative individuals, at different dilutions of both purified, and unpurified fusion proteins and blood samples. Visible agglutination was observed in all seropositive blood samples.

The fusion proteins did not agglutinate erythrocytes from seronegative blood donors used as negative controls.

### EXAMPLES:

These examples pretend to illustrate the invention, but not to limit its scope.

### EXAMPLE 1.-

### Amplification of the genes that encode for the F41 and PapG adhesins of piliated E. coli bacteria, using the Polymerase Chain Reaction (PCR). Cloning into bacterial vectors and base sequencing.

### Procedure (a). DNA isolation.-

Total DNA was purified (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY) from ETEC 1593 (K99-, F41+) for the F41 adhesin, and from E. coli J96 for the PapG.

### Procedure (b). DNA Amplification.-

PCR was used for specific amplification of the F41 and papG adhesin genes, from the first aminoacid reported for the mature proteins, to the respective stop codons. The synthetic oligonucleotides were designed based on reported sequences (Lund, B., Lindberg, F., Marklund, B-I. y Normark, S. 1987. Proc. Natl. Acad. Sci.USA. 84. 5898-5902, Anderson, D. y Moseley, S. 1988. J Bacteriol. 170. 4890-4898), and included convenient restriction end sites for cloning (Table 1). PCR conditions were separately optimized for each adhesin gene.

**Table 1**

| Synthetic oligonucleotides used for the specific PCR amplification of the genes encoding for the F41 and Pap G adhesins. |
|---|
| Oligo No. 1.- 5' End Pap G gene. Cla I. |
| 5' ...CCATCGATG AAA AAA TGG TTC CCT GCT TTT TTA.. |
| Oligo No. 2.- 5' End Pap G gene. EcoR I/ Nco I. |
| 5' ...CG GAATTCT T CCATGG GA TGG CAC AAT GTC ATG TTT TAT GC.. |
| Oligo No. 3.- 3' End pap G gene. Eco RV. Antisense. |
| 5' ...GG GATATC GGG GAA ACT CAG AAC CAT AGT C.. |
| Oligo No. 4.- 5' End F41 gene. Cla I. |
| 5' ... CCATCGATG AAA AAG ACT CTG ATT GCA CTG GCT G.. |
| Oligo No. 5.- 5' End F41 gene. EcoRI/ Nco I. |
| 5' ...CG GAATTCT T CCATGG CT GCT GAT TGG ACG GAA GGT C.. |
| Oligo No.6.- 3' End F41 gene. EcoRV. Antisense |
| 5' ...GG GATATC ACT TAT AAT AAC GGT GAT AGT CAC.. |
| Note: The restriction sites are underlined. |

The amplified DNA fragments were purified from low melting point agarose gels using saturated phenol extraction, and digested with the proper restriction enzymes for the cloning into the expression vectors (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY).

### Procedure (c). Cloning of the amplified adhesin genes into the plasmid vector designed for periplasmic expression.-

The vector pPACIB.7+ (see 30 for related vector) was digested EcoRI-EcoRV (or Cla I-EcoRV), and ligated to digested PCR inserts in two separate reactions: (1) pPACIB.7+ + Pap G, and (2) pPACIB.7+ + F41. Ligation products were used for the transformation of competent E. coli cells (XL-1 Blue), that were then plated on semisolid selective medium and grown at 37°C (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY).

The recombinant plasmids were selected after the purification of plasmid DNA from a number of bacterial colonies, and their digestion by restriction enzymes to check for the expected ligation product (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY). In this analysis, a 3 kb band was obtained for the linearized pPACIB.7+ vector, and 1 kb and 630 bp bands for the Pap G and the F41 genes, respectively. At least three clones for each adhesin were selected and sequenced by conventional methods (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY).

The sequencing was performed on the denatured of the plasmid DNA double-helix chains by 10 N NaOH treatment, and according to the Sanger method (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY). The cloned genes were sequenced in both directions using primers specifically designed for this purpose that hybridize outside from the cloning sites of the plasmid pPACIB.7+. The sequences of the two amplified and cloned adhesin genes matched those reported in the literature for F41 and Pap G (Lund, B., Lindberg, F., Marklund, B-I. y Normark, S. 1987. Proc. Natl. Acad. Sci.USA. 84. 5898-5902, Anderson, D. y Moseley, S. 1988. J Bacteriol. 170. 4890-4898)

### Procedure (d). Cloning the adhesin amplified genes into the plasmid vector designed for intracellular expression pQE 60.-

The vector pQE 60 (Qiagen) was digested NcoI-BamHI. The pPACIB.7+ vectors containing each adhesin insert were also digested NcoI-BamHI. The DNA fragments of 1 kb (Pap G) and 670 bp (F41) were purified from low melting point agarose gels and ligated into the previously prepared pQE vector. Ligation products were used for the transformation of competent E. coli cells (XL-1 Blue), that were then plated on semisolid selective medium and grown at 37°C for 16 hours (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY).

The recombinant plasmids were selected after the purification of plasmid DNA from a number of bacterial colonies, and their digestion with restriction enzymes to check for the expected ligation product (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY), that is, a band corresponding with the linearized vector with a 3.5 kb size for the pQE 60 vector, and 1 kb and 630 bp bands corresponding to the papG and the F41 genes, respectively.

### Procedure (e). Cloning the adhesin amplified genes into the plasmid vector designed for intracellular expression pPACIB.9+.-

The vector pPACIB.9+ was digested EcoRI-BamHI. The pPACIB.7+ vectors containing each adhesin insert were also digested EcoRI-BamHI. The DNA fragments of 1 kb (Pap G) and 670 bp (F41) were purified from low melting point agarose gels and ligated into the previously prepared vector. Ligation products were used for the transformation of competent E. coli cells (XL-1 Blue), that were then plated on semisolid selective medium and grown at 37°C for 16 hours (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY).

### EXAMPLE 2.-

### Construction of the adhesin-HIV-1 peptide fusion proteins, and bacterial expression.

### Procedure (a). Construction of the fused genes.-

The two oligonucleotides (Table 2) encoding for the 15 aminoacid gp41 HIV-1 peptide were annealed at 70°C and phosphorilated using PNK (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY). The plasmids pPAPs, pF41s, pPAPi, and pF41i were digested BamHI-ApaI and ligated with the assembled DNA. Ligation products were used to transform competent E. coli cells (XL-1 Blue), that were then plated on semisolid selective medium and grown at 37°C for 16 hours (Sambrook, J., Fritisch, E.F. y Maniatis, T. 1989. En; Molecular Cloning. A laboratory Manual. Sec. Ed. Cold Spring Harbor Laboratory Press, Cold Spring Harbor, NY).

The recombinant vectors were selected after the purification of the plasmid DNA from a number of bacterial colonies, and their digestion by restriction (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY). The clones with expected restriction patterns were sequenced as described in Example 1.

**Table 2**

| Synthetic oligonucleotides for the assembly of the gene encoding for the 16 aminoacid HIV-1 peptide. |
|---|
| Oligo No.7 . HIV-1 peptide, sense |
| 5' ...GATCC GAT CAG CAG CTG CTG GGC ATC TGG GGC TGC AGC GGT AAA CTG TAT TGC GGGCC... |
| Oligo No. 8. HIV-1 peptide, antisense |
| 5' ...C GCA ATA CAG TTT ACC GCT GCA GCC CCA GAT GCC CAG CAG CTG CTG ATC G... |

### Procedure (b). Expression of the fused genes in E. coli.-

The same bacterial strains assayed for the expression of the adhesin genes were assayed for the four genetic constructions described in Procedure (a). Basically, the recombinant bacteria were grown overnight at 37°C in liquid medium (LB), supplemented with ampicillin (Sambrook, J., Fritisch, E.F. y Maniatis, T. 1989. En: Molecular Cloning. A laboratory Manual. Sec. Ed. Cold Spring Harbor Laboratory Press, Cold Spring Harbor, NY). Fresh cultures with LB and ampicillin were started, and incubated for three hours at 37°C. Expression of the proteins of interest was induced by adding to the cultures beta-indoleacrylic acid for the pPACIB expression constructions, or IPTG to the pQE expression constructions. The analysis of bacterial samples in 15% SDS-polyacrylamide gels indicated that under these conditions new proteins corresponding with the expected molecular sizes were expressed. Expressions were confirmed by Western blot analysis using a specific human antiserum for the HIV-1 peptide, pre-adsorbed against E. coli proteins to prevent non specific background.

### EXAMPLE 3

### Cloning of the fusion protein into the Pichia pastoris expression vector pHIL-S1, and generation of recombinant yeast strains.

### Procedure (a). DNA amplification.-

PCR with oligonucleotides reported in Table III was done on the F41-HIV and PapG-HIV fusion protein genes cloned in the expression bacterial vectors described above, for the introduction of required restriction sites.

**Table 3**

| Synthetic oligonucleotides for the cloning on the yeast vector. |
|---|
| Oligo No. 9. PapG Gene. EcoRI sense. |
| 5'...CG GAATTCT CCA TGG GAT GGC ACA ATG TCA TGT TTT ATG C... |
| Oligo No. 10. F41 Gene. EcoRI sense. |
| 5'...CG GAATTCT CCA TGG CTG CTG ATT GGA CGG AAG GTC.. |
| Oligo No. 11. T4 Terminator antisense |
| 5'...GGT CAT TCA AAA GGT CAT CCA C.. |

### Procedure (b). Cloning of the F41-HIV and PapG-HIV genes into the pHIL-S1 vector.-

The pHIL-S1 vector was digested EcoRI-BamHI and ligated to digested PCR inserts in two separate reactions: (A) pHIL-S1 + F41-HIV (B) pHIL-S1 + PapG-HIV.

The ligation products were used for the transformation of competent Top 10 cells that were plated on semisolid selective medium and grown at 37°C, overnight. Ten ampicillin resistant colonies were selected and inoculated into LB medium with 50 ug/ml of ampicillin, and grown overnight at 37°C with shaking.

The miniprep isolated plasmid DNAs were digested by restriction enzymes to check for the expected ligation product. A minimum of 3 different ones for each fusion protein gene were selected and sequenced using the 5' AOX1 and 3' AOX1 sequencing primers.

### Procedure (c). Transformation of GS 115 Pichia pastoris strain.-

The plasmids bearing F41-HIV and PapG-HIV genes in the correct orientation for expression were prepared for Pichia pastoris transformation by digesting at the unique Stu I site, and extracted with phenol:chloroform:isoamyl alcohol (24:25:1). The precipitated

DNA pellet was suspended in 10 ul TE. GS 115 cells were grown at 30°C overnight and prepared for electroporation. Ten ul of DNA of each construction were used to transform
by electroporation (BioRad Gene Pulser). The electroporated cells were spread on Minimal Dextrose (MD) medium plates and grown at 30°C until the colonies appeared.

### Procedure (d). Screening and confirmation of integration of His+ transformants for Mut+ and MutS strains.-

Transformation of GS 115 with Stu I linearized pHIL-S1 constructs favors recombination at the His4 locus. By replica plating on MD versus Minimal Methanol (MM) plates, Mut+ and MutS transformants were identified. Ten transformants of each construction were tested and all were Mut+. Confirmation of integration was done by colony Hot Start PCR.. The 5' AOX1 and 3'AOX1 primers amplified the cloned bands. Five, and seven out of 10 studied clones for each PapG-HIV and F41-HTV, respectively, showed the expected 1 kb and 700 bp bands indicative of integration.

### EXAMPLE 4.-

### Expression of the adhesin-HIV peptide fusion proteins in Pichia pastoris.-

### Procedure (a). Growth of the His+ Mut+ recombinants in buffered glycerol medium (BMGY) and induction.-

The 5 and 7 PCR positive transformants of F41-HIV and PapG-HIV were grown in 10 ml of BMGY medium at 30°C to final ODs of 2-6.

The cells were harvested and suspended with methanol medium to 1 O, and incubated at 30°C with shaking . Samples were taken for analysis every 24 hours, for 4 days. Pure methanol was added to a final concentration
of 0.5% every 24 hours to maintain induction

### Procedure (b). Analysis of culture supernatant for fusion proteins using SDS-PAGE, and Western blot.-

One ml of the expression culture at each time point was centrifuged and the supernatant transferred to a separate tube for protein expression analysis, ater 10 fold concentration with 100% TCA The analysis of samples in 15% SDS-polyacrylamide gels indicated that under these conditions new proteins corresponding with the expected molecular sizes were expressed. Expressions were confirmed by Western blot analysis using a specific human antiserum for the HIV-1 peptide.

### EXAMPLE 5.-

### Production of the fusion proteins from the bacterial cultures, and assays for specificity of bacterial and yeast fusion proteins with human erythrocytes.

### Procedure (a). Extraction of the intracellular bacterial fusion proteins (pPAP HIVi and pF41 HIVi).-

The recombinant bacteria carrying the plasmids designed for cytoplasmic expression were subjected to an induction process, as described above, followed by lysis by ultrasound, and separation of the soluble and insoluble fractions. It was shown by Western blot that both recombinant fusion proteins remained associated to the insoluble cell fraction. The specific proteins were extracted from the rest of the contaminants with a chaothropic solubilizing agent, and submitted to dialysis against 0.1 M sodium phosphate, 0.5 M NaCl, pH 8.0 (coupling solution), before purification.

### Procedure (b). Extraction of the periplasmic bacterial fusion proteins (pPAP HIVs and pF41 HIVs).-

For the extraction of the fusion proteins expressed in the periplasm, the cells containing the recombinant products were frozen and thawed for three consecutive times, centrifuged, the supernatant containing the fusion proteins submitted to dialysis against 0.1 M sodium phosphate, 0.5 M NaCl, pH 8.0 (coupling solution), and saved for purification.

### Procedure (c). Purification of the fusion proteins using immobilized metal ion affinity chromatography (IMAC).-

For purification we took advantage of the histidine domains present in the recombinant proteins. These sequences confer proteins with a very high affinity for certain metal ions (for example, Zn+2, Cu+2, Ni+2) that can be chelated to different chromatographic supports, rendering purification easy and reproducible procedure.

The fusion proteins obtained as described in procedures (a) and (b), were directly applied to a Sepharose-IDA-Cu +2 or Ni+2 matrixes. Once the couplings were made, the gels were first washed with 10 times their volume using coupling buffer adjusted to pH 6.3, followed by a similar wash with the solution adjusted to pH 5.0 (for the elution of E. coli contaminant proteins). The elution of the fusion proteins was made at pH 4.0 with the same solution, with immediate neutralization of eluates with Tris base.

### Procedure (d). Renaturing of the fusion bacterial proteins expressed in the cytoplasm (pPAP HIVi and pF41 HIVi).-

The cytoplasmic fusion proteins pPAP HIVi and pF41 HIVi were renatured after purification by extensive dialysis at 4°C, using a redox buffer designed to the effect, after a total reduction with DTT. The protein preparations were concentrated by ultrafiltration after removal of aggregates by centrifugation.

### Procedure (e) Agglutination assay using human blood erythrocytes.

The purified bacterial fusion proteins, and the yeast culture supernatants, were tested for agglutination of human erythrocytes. Successive double dilutions in PBS of the fusion proteins (estimated initial concentration of 100 ug/ml) were made in the wells of microtiter plates, to a final volume of 50 ul/well. After that, 50 ul of a 1% (vol/vol) suspension of O+ human erythrocytes in: (a) a specific antiserum against each adhesin, (b) a human serum containing antibodies specific for the HIV-1 peptide, or (c) an unrelated antiserum, were added per well. The plates were allowed to stand for 1-5 minutes at room temperature before they were visually read. It was found that the recombinant fusion proteins visibly agglutinated erythrocytes in the correct samples (those containing an antiserum against the specific adhesin, or human serum antibodies specific for the HIV-1 peptide).

### Procedure (f). Agglutination assay using direct human blood.-

Successive double dilutions in PBS of the fusion protein solutions (estimated initial concentration of 100 ug/ml) were made in the wells of microtiter plates, to a final volume of 50 ul/well. After that, 50 ul of dilutions of fresh vein blood in PBS from HIV-1 seropositive or seronegative individuals were added per well. The plates were allowed to stand for 1-5 minutes at room temperature before they were visually read. All the fusion protein preparations showed a specific hemagglutinating activity (only in samples from seropositive individuals) at defined dilutions of both protein and blood.

## Claims

1. A fusion protein composed in part of a bacterial adhesin, able to specifically bind to the surface of human red blood cells, and in part of a polypeptide able to be recognized as an antigen.

2. A fusion protein as described in Claim 1 in which its red blood cell binding moiety is restricted to only one or several of the original domains of a bacterial adhesin molecule.

3. A fusion protein as described in Claims 1 and 2 in which the red blood cell binding moiety was originated, or further modified, using recombinant DNA technology.

4. A fusion protein as described in Claims 1, in which the non adhesin part of the molecule is not an antigen, but one or more antibody binding sites.

5. A fusion protein as described in Claims 1 to 4, created using recombinant DNA technology, or by chemical coupling of individually produced molecules.

6. A fusion protein as described in Claims 1 to 5, useful for the development of a diagnostic test in which the end result takes the form of a red blood cell agglutination reaction.

7. A fusion protein as described in Claims 6, in which the end result takes the form of a red blood cell agglutination reaction, that can be read visually, or using any appropriate equipment.

8. The use of the fusion protein described in Claims 1 to 7 in the production of a reagent useful for the development of red blood cells agglutination assays.
